# EUROPEAN PATENT APPLICATION

(11) **EP 4 600 294 A1**
(43) Date of publication of application: **13.08.2025**
(21) Application number: 24156882.3
(22) Date of filing: 09.02.2024
(51) Int. Cl.: C08J 11/06, C08L 67/04, C08L 101/12, A61B 90/18, A61L 15/12, A61N 5/10, B29C 33/38

(54) **THERMOPLASTIC MATERIAL FOR IMMOBILIZATION PURPOSES**

(71) Applicant: Orfit Industries N.V., 2110 Wijnegem (BE)
(72) Inventor: HUSSEIN, Naji, 2110 Wijnegem (BE); CUYPERS, Steven, 2110 Wijnegem (BE)
(74) Representative: V.O.

(57) **Abstract**

A composition for use in an immobilization device for immobilizing at least a portion of a body part comprising, based on total weight of the composition,
a. 55 to 90 wt%, preferably 60 to 80 wt%, more preferably 65 to 75 wt% of a first polymer and/or a first regrind by-product thermoplastic material derived from such a first polymer which is not chemically crosslinked, with a melting temperature of below 100°C, preferably of between 40 and 75°C;
b. 5 to 35 wt%, preferably 10 to 30 wt%, more preferably 15 to 20 wt% of a second regrind by-product thermoplastic material derived from such a first polymer which is at least partially chemically crosslinked;
c. optionally 2.5 to 15 wt%, preferably 5 to 10 wt% of a thermoplastic elastomer;
d. 0 to 5 wt% of total additives.

## Description

The invention relates to a composition and thermoplastic material for use in an immobilization device which is provided to cover at least a portion of a human or animal body part that needs to be fixed, supported and/or immobilized. The invention furthermore relates to a method for manufacturing such thermoplastic material and immobilization device.

The use of fixation and immobilization devices for immobilizing a part of a body has become a well-known technology in applications such as orthotics and prosthetics, physical rehabilitation, radiation therapy and diagnostic imaging. Those applications require that the immobilization device is mouldable at activation temperature directly on a patient, that the immobilization device shows good mechanical properties and surface finishing and is light and comfortable to the patient.

Immobilization devices know a wide application in radiation therapy and diagnostic imaging for the purpose of immobilizing a body part in a fixed and reproducible position with respect to an irradiation source in a wide range of equipment, inter alia, an accelerator, NMR, MRI, CT, etc. In these applications a correct positioning of the body part with respect to the radiation source is important, in order to ensure that the radiation is directed at the body part to be treated and the risk of irradiating surrounding healthy issue is kept to a minimum. A reproducible positioning is of utmost importance in fractionated therapy, in which a segment, i.e. one or more portions of a body part are repeatedly subjected to irradiation, with intermittent time intervals between subsequent irradiation sessions. The stability of the immobilization device plays an important role in the above-mentioned applications, by which is meant that the ability of the body part to be immobilized, to be moved or displaced after having been immobilized, is limited to less than a few mm.

In radiation therapy and diagnostic imaging often use is made of a bench or table, to which the patient is positioned in a supine or prone position, while the segment of the body part to be treated is immobilized at the desired position in a desired configuration. In order to enable achieving the desired immobilization a wide variety of masks have been developed which are placed over a portion of the body, for example the head, a portion of the shoulders and a portion of the chest of the patient, and are connected to the bench or table. In order to guarantee an optimal fit with the body part to be immobilized, to ensure that the inner surface of the sheet of the thermoplastic material fits as close as possible to the outer contours of the body part to be immobilized, such as head, shoulders and/or chest and the intended immobilization is achieved, the mask is formed directly on the patient's body. Fastening of the mask to the table is made possible by the presence of one or more connecting profiles along the edge of the mask which can be connected to a corresponding profile on the table.

To produce fixation or immobilization devices, which are suitable for use in the above described applications, usually use is made of a sheet shaped thermoplastic material, which is moulded to conform as good as possible to the body part that is to be immobilized. Over the years, continuous development has been going on towards materials, which meet specific requirements of the envisaged application. The use of sheets of thermoplastic materials which may be directly moulded to the body part to be immobilized has achieved significant attention, as this permits achieving immobilization with the highest accuracy, where the size and shape of the immobilization device may be directly adapted to each individual patient in the position in which the body part is to be immobilized, and it may be adapted in the course of time by re-moulding the immobilization device. To permit this direct moulding, the thermoplastic material should have a melting temperature which is sufficiently low to be sustained by the body. Besides that, the material should have sufficient formability and elasticity in the molten state, for a period of time which is sufficiently long to permit moulding, but not too long to save clinical time and minimize the risk to deformation after moulding has been completed. A thermoplastic material which is particularly suitable for direct moulding to a body part is commonly based on polycaprolactone.

When molding the immobilization device directly to the body part to be immobilized, for example as a mask to the patient's face and head, the sheet or template is heated (e.g. in a warm water bath) in order to achieve that the sheet becomes flexible and moldable and can be shaped into the desired form. The heated sheet or template is positioned to the face or head, shaped and stretched to conform to the contours of the face or head as close as possible, with the purpose of incorporating into the shape of the template many details of the face or head, such as, for example, nose, mouth, eyes, ears, protruding bones etc. This shaping typically takes some time and therefore it is important that the material of the sheet or template keeps its flexibility and formability for a period of time which is sufficiently long to permit the necessary shaping to be carried out. Therefore, on the one hand the crystallisation/solidification rate of the template material should be sufficiently slow in order to have a sufficiently long molding time available for adequate, detailed molding of the template or sheet. On the other hand it is important that the molding time is sufficiently short and that the crystallization/solidification rate of the thermoplastic material is sufficiently fast to reduce patient waiting times during cooling and hardening of the mask after the molding has been finished. Besides this, the direct shaping of the template not only involves positioning of the template on top of the body part, but also involves a significant amount of stretching and moulding of the thermoplastic material around the body part while the thermoplastic material contacts the body part. The stretching and shaping is sensed by the patient as particularly unpleasant, frightening and claustrophobic. After the moulding of the mask has been finished, the mask is left to cool and stiffen to a rigid structure with a desired positioning and fixation ability to hold the head, or other body part stationary in a desired position.

In order to achieve a correct molding/shaping of the mask, the viscosity or melt strength of the thermoplastic material after being softened (normally by heating), should be high enough to allow handling, stretching and shaping of the thermoplastic mask around the body part. A low viscosity will result in an excessive stretching of the mask shape due to the high material flow. A high melt strength or viscosity will result in high resistance of the thermoplastic to stretching. In both cases the material molding and shaping of thermoplastic masks around the body will be impossible.

In order to achieve the right melt strength, a crosslinking step of the thermoplastic is commonly performed. The thermoplastic sheet containing the polymer and a suitable crosslinking agent is in most cases at least partially chemically-crosslinked to crosslink the polymer material by forming covalent bonds within, between and through the various polymer chains of the composition. Crosslinking may be achieved using any of the suitable techniques known to the skilled person to achieve chemical crosslinking, which permit to control the degree of crosslinking and the mechanical properties of the polymer sheet after crosslinking has been carried out.

Suitable techniques to achieve chemical crosslinking include crosslinking using a peroxide compound (see GB 8417872), in which case crosslinking may be initiated by heating the thermoplastic composition, and crosslinking by exposing the thermoplastic sheet to irradiation, for example UV radiation (see EP 3069697, EP 3752109), electron beam radiation (see US 4240415, JP 2005008892), or γ-radiation (see US 11364675, US 8859691, US 11364675), but any other technique considered suitable by the skilled person may be used as well.

γ-radiation crosslinking is usually carried out in expensive devices, especially provided for that purpose, normally not present at the production site of the template, thereby making the production process slow and complex and inflexible. When use is made of γ-radiation in general an energy dose γ-radiation of between 1 and 100 KGy will suffice to achieve the desired degree of chemical crosslinking, preferably between 1 and 60, more preferably between 1 and 50 Kgy. A more advantageous technique to achieve chemical crosslinking involves subjecting the thermoplastic material to UV radiation. This type of crosslinking presents the advantage that it may be carried out on each individual template or sheet on-site where the template is produced or intended for use. Because of the smaller energy provided by a UV-source, UV radiation permits to easily control the degree of crosslinking, taking into account the intended application. The duration of the irradiation with UV, and the energy of the UV radiation source, may be varied within wide limits, and is preferably chosen such that the desired degree of crosslinking is achieved without degradation of the polymer sheet to occur. Generally, the sheet is exposed to UV radiation preferably for a period of 30 seconds to 1 hour at a radiation power of 10 watts to about 500 watts. The skilled person is capable of matching the radiation intensity and duration to the intended degree of crosslinking taking into account the thickness of the polymer sheet, the transparency of the sheet to UV radiation and the amount of photo-initiator present in the sheet.

A higher degree of crosslinking usually leads to a thermoplastic polymer sheet with a higher toughness and rigidity, a higher modulus of elasticity in the molten state and less plasticity and less elasticity in the molten or soften state. When forming such a sheet in the molten or soften state in an immobilization element on the body to be immobilized, this usually leads to a formed sheet with a higher thickness and better stability. A lower degree of crosslinking usually leads to a polymer with a lower toughness and rigidity, a lower modulus of elasticity in the molten state, a higher stretchability in the molten or soften state. When forming such a sheet in the molten or soften state in an immobilisation element on the body to be immobilized, this usually leads to a formed sheet of a smaller thickness. Properties such as modulus of elasticity and elasticity of the polymer sheet in the molten or softened state can thus be controlled by controlling the degree of crosslinking.

The polymer sheet prior to the exposure to irradiation is preferably cut into a desired shape although cutting may also take place after exposure to irradiation. This offers the advantage that the waste or by-product material is not crosslinked and can be recovered for recycling. Perforation of the sheet may be carried out before or after the exposure to irradiation. Usually perforation is carried out prior to exposure to irradiation to better achieve uniformity in the crosslinking and to allow recycling of perforation material. An average of up to 30 % of the material is lost as waste or by-product when cutting and perforating.

The extra step of chemical crosslinking which is currently used in the standard production process is costly as it requires heavier equipment. An alternative to chemical crosslinking may be physical crosslinking.

Thermoresponsive materials based on blending polycaprolactone with a second polymer e.g. an amorphous TPU are described in numerous publications. See, for example, by Xin Jing, Hao-Yang Mi, Han-Xiong Huang, Lih-Sheng Turng under the title "Shape memory thermoplastic polyurethane (TPU)/poly(e-caprolactone) (PCL) blends as self-knotting structures" in Journal of the Mechanical Behavior of Biomedical Materials 64 (2016) p 94-103. Also, by Nasim Sabahi, Iman Roohani, Chun H. Wang, Ehsan Farajzadeh, Xiaopeng Li under the title "Thermoplastic polyurethane-based shape memory polymers with potential biomedical application: The effect of TPU soft-segment on shape memory effect and cytocompatibility" in Polymer 283 (2023), 126189. In these materials the crystalline phase of polycaprolactone is responsible for the thermo-shaping characteristic while the amorphous phase of TPU is responsible for the fixable net points and shape recovery stage of material (physical crosslinking points).

These materials are physically crosslinked whereby thus the additional step needed for chemically crosslinking is avoided. It is important that the immobilisation masks made out of such compositions can be activated at relatively low temperature (generally 65°C) and has sufficient working time or setting time to allow the mask molding/shaping before full mask solidification. Moreover the shrinkage due to the crystallization of polycaprolactone should be as low as possible to avoid patient discomfort.

There is thus a need for a thermoplastic sheet that can be produced without the extra step of chemical crosslinking and that can also be used in the more demanding application of immobilization devices for use in radiation therapy and diagnostic imaging. Such a sheet would offer more flexibility in the production process as no heavy equipment is needed for the gamma or UV crosslinking. It will also make the production cycle shorter, easier and cheaper.

Further there is also a need to develop a thermoplastic material that provides the right balance between melt strength, stretchability, easiness of shape forming, solidification speed, shape fixation and patient comfort and stability and that is suitable for use in immobilization devices without requiring chemical crosslinking,

Further there is also a need for a product that is more sustainable and environmentally friendly.

It was found that a novel composition could be used to produce a thermoplastic sheet having the right balance of properties as mentioned above, that does not require a chemical crosslinking step, that is suitable for use as immobilization device in radiation therapy and diagnostic imaging applications and that is sustainable and environmentally friendly.

Accordingly the invention provides a composition comprising, based on total weight of the composition,
a. 55 to 90 wt%, preferably 60 to 80 wt%, more preferably 65 to 75 wt% of a first polymer and/or a first regrind by-product thermoplastic material derived from such a first polymer which is not chemically crosslinked, said first polymer and/or said first regrind having a melting temperature of below 100°C, preferably of between 40 and 75°C;
b. 5 to 35 wt%, preferably 10 to 30 wt%, more preferably 15 to 20 wt% of a second regrind by-product thermoplastic material derived from such a first polymer, said by-product being at least partially chemically crosslinked;
c. optionally 2.5 to 15 wt%, preferably 5 to 10 wt% of a thermoplastic elastomer;
d. 0 to 5 wt% of total additives.

The composition according to the invention through careful selection of its components and amounts thereof allows to produce a thermoplastic material that has the right balance of properties so that it can be used as immobilization device particularly in radiation therapy and diagnostic imaging, in particular with respect to patient comfort and fixation stability. Further a chemical crosslinking step is not needed in order to produce the thermoplastic material which simplifies the production process. Due to the use of regrind by-product material, a low-cost and sustainable, environmentally friendly product is provided without detrimentally affecting the required properties whereas previous attempts to use by-product material in said applications led to tighter and uncomfortable masks.

The composition according to the invention was tuned in such a manner that the material can be easily molded around the body part to be immobilized and yet the thermoplastic rigidity after solidification is high enough to ensure a patient immobilization equivalent to the one offered by high precision thermoplastic chemically-crosslinked masks. Despite the relatively high stability of the shaped masks produced from the composition of the present invention, the thermoplastic material does not shrink significantly. Consequently they are considered as highly comfortable immobilization devices.

For the purpose of clarity and a concise description, features are described herein as part of the same or separate embodiments, however, it will be appreciated that the scope of the invention may include embodiments having combinations of all or some of the features described.

As used herein, the singular forms "a", "an" and "the" are intended to include the plural forms as well. The term " or" includes any and all combinations of one or more of the associated listed items, unless the context clearly indicates otherwise (e.g. if an "either ....or" construction is used). It will be understood that the terms "comprises" and "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise.

As is used herein, the term "wt %", or "weight percentage", or "percentage by weight", refers to the mass fraction of a substance in a mixture divided by the total mass of said mixture, expressed as a percentage between 0 and 100.

As is used herein the term "regrind by-product thermoplastic material" refers to by-product from a thermoplastic material that has been chopped and regrinded. By-product can refer to by-product generated during the production process of the thermoplastic material e.g. by perforating some parts of the thermoplastic material or by cutting it into a desired shape. By-product can also refer to thermoplastic material that has been used such as used immobilisation devices. The regrinding can take place in any suitable apparatus such as a shredder. The material is regrinded into a size that matches the new or virgin material being processed. In the case of the present invention this is usually below 5 mm, preferably around 3 mm, to allow feeding of the regrinding material into an extruder. Various shapes of the regrind may be possible, also irregular shapes.

The invention provides a composition comprising at least a first polymer and/or a first regrind by-product thermoplastic material, a second regrind by-product thermoplastic material, optionally a thermoplastic elastomer and optionally additives.

A first polymer as comprised in a composition according to the invention is a thermoplastic polymer with a low melting temperature (generally below 100°C or even below 75°C). The melting temperature of said first polymer can be measured by Differential Scanning Calorimetry (DSC). The measured melting point corresponds to the peak observed in a DSC heating curve, at a heating rate of 10°C/minute. A first heating scan is usually done in order to delete the previous thermal history of the sample. After a cooling ramp at 10°C /min to -80°C a second heating scan is also performed at the same rate (10°C/min). Depending on the thermal history of the sample, the melting temperature measured during the first heating can be few degrees different from the one measured during the second heating scan. The inherent melting temperature (second heating peaks) are the values that correspond to the melting temperatures mentioned in the present description. Preferably the melting temperature, measured via DSC, of said first polymer is between 40 and 75°C, more preferably between 50 and 70°C, most preferably between 51 and 61°C, as these temperatures may be supported by the human and animal body and permit direct molding to the body.

Examples of thermoplastic polymers with such melting temperatures are thermoplastic polyurethanes, thermoplastic polyesters and thermoplastic polyolefins. Only one of said polymers can be used or a blend of two or more of them. Examples of suitable thermoplastic polyolefins include ethylene copolymers, ethylene vinyl acetate copolymers, propylene copolymers, ethylene-propylene copolymers or poly cis-isoprene. Examples of suitable thermoplastic polyesters include polycaprolactone, polymeric fatty acid esters, butylene adipate terephthalate copolymers, succinate-butylene adipate copolymers. Preferred first polymers are selected from the group consisting of polycaprolactone (PCL), thermoplastic polyurethane (TPU), polyalkenamer and poly cis-isoprene. Preferably, the first polymer of a composition according to the invention is a polycaprolactone (PCL) polymer, most preferably ε-polycaprolactone. ε-polycaprolactone is particularly preferred because it has a low melting point, below 75°C and particularly between 40°C and 70°C (measured by DSC), shows good moulding properties and has sufficiently long working time before it solidifies in order to allow direct molding on the body. If so desired, the ε-polycaprolactone can be used in a mixture with another thermoplastic, for example thermoplastic polyurethane.

Particularly preferred is a first polymer (in particular ε-polycaprolactone) with a number average molecular weight Mn of at least 10.000 g/mole, more preferably at least 20.000 g/mole, most preferably at least 40.000 g/mole, in particular at least 50.000 g/mole. The first polymer of the invention will generally have a number average molecular weight Mn of maximum 100.000 g/mole, preferably maximum 90.000 g/mole. A first polymer with a molecular weight above 100.000 g/mole may lead to slower crystallisation rates and reduce the moldability of the thermoplastic material in the molten state, thereby compromising the ability of the material to be shaped in the molten state and adversely affecting patient comfort. However crystallization can be speeded up by adding nucleating agents. PCL with varying molecular weights are commercially available from the company Ingevity under the trade designation CAPA.

In addition to the first polymer or instead of the first polymer, a first regrind by-product thermoplastic material with a melting temperature of below 100°C is used in the composition of the present invention. This first regrind originates from by-product material of a thermoplastic material that is derived from a first polymer as described above, said by-product material not being chemically crosslinked. For example, the first regrind can be by-product material from the production of a thermoplastic material derived from such a first polymer prior to it being crosslinked such as edge strips, leftover from cutting the sheets into precuts and perforation by-product since perforation is usually done prior to chemical crosslinking. Alternatively said first regrind can also be by-product material from a physically crosslinked thermoplastic material.

A composition according to the invention comprises 55 to 90 wt%, preferably 60 to 80 wt%, most preferably 65 to 75 wt%, of a first polymer and/or a first regrind. Solely first polymer or solely first regrind may be used, or any mixture thereof. For cost reasons using a majority or solely first regrind may be preferred. In particular, good results have been obtained with PCL (namely Polycaprolactone CAPA 6500, which has a molecular weight of 50,000 g/mole and a bending modulus of 411 MPa according to ASTM D790).

The molecular weight Mn of a compound can, for example, be determined by gel permeation chromatography (GPC) according to DIN 55672-1, using THF as eluent. Unless otherwise indicated, the molecular weights listed are those that have been determined by GPC.

The composition of the present invention further comprises a second regrind by-product thermoplastic material. This second regrind originates from by-product material of a thermoplastic material that is derived from a first polymer as described above, said by-product material being at least partially chemically crosslinked. The term "at least partially chemically crosslinked" means that the by-product material has been subjected to some degree of chemical crosslinking by any of the abovementioned chemical crosslinking methods, in particular crosslinking by irradiation. The degree of crosslinking will depend on the amount of crosslinker present, the thickness of the thermoplastic material and the duration and energy of irradiation. The second regrind can be by-product material from the production of a thermoplastic material derived from such a first polymer after it being chemically crosslinked such as cutting by-product. Alternatively said second regrind can also be by-product in the form of a used thermoplastic material such as a used immobilization device. Mixtures of different types of second regrind can be used as well. In a preferred embodiment the second regrind contains by-product material from thermoplastic material that contains nanofillers (e.g. as described in WO 2011/113473), in particular nanoclay; such regrind material can act as melt strength enhancer and adjusts the working time.

A composition according to the invention comprises 5 to 35 wt%, preferably 10 to 30 wt%, most preferably 15 to 20 wt%, of a second regrind. The amount of second regrind that is needed in the formulation will depend on the level of crosslinking of the second regrind, with a higher degree of crosslinking a lower amount of second regrind being needed. The degree of crosslinking can indirectly be determined through the RTS value (as described below), the lower the RTS value the higher the degree of crosslinking is. So, e.g, if as second regrind a by-product is used that has a relatively low crosslinking density (e.g. a RTS of 100 to 140 cm) then a higher amount of said second regrind is used in the composition (e.g. about 30 wt%). In case as second regrind a by-product of high crosslinking density (e.g. RTS of 40 to 70 cm) is used then the amount thereof is usually about 15 wt%.

A thermoplastic elastomer (TPE) as comprised in a composition according to the invention can be any polymer exhibiting elastic, flexible or rubber-like properties at room temperature and that can be processed with plastic manufacturing techniques such as extrusion, injection and blow moulding.

Said thermoplastic elastomer is preferably a binary mixture of soft segments and hard segments where the soft segments are rubbery as their glass transition temperature is below room temperature.

In one embodiment the hard segment of the TPE is an amorphous homopolymer that has a glass transition temperature above room temperature. In another embodiment the hard segment of the TPE is a semi-crystalline polymer.

Said thermoplastic elastomer preferably has a shore A hardness of up to 92, preferably from 30 to 80 and is preferably compatible with the first polymer (e.g. PCL). Suitable thermoplastic elastomers for use according to the present invention include Thermoplastic olefinic elastomers (TPOs), thermoplastic polyurethane elastomers (TPU) and so-called Thermoplastic styrenic elastomers (TPS) such as polystyrene-butadiene or polystyrene-isoprene copolymers, generally with a styrene content of up to 60 wt%, preferably between 15 and 50 wt%, more preferably between 30 and 35 wt%, wherein the styrene phase is partially miscible with PCL and the rubber phase (butadiene or isoprene) improves the toughness (comfort)).

The thermoplastic elastomer preferably has a molecular weight above 100,000 g/mol, preferably above 150,000 g/mol, more preferably about 170,000 g/mol. The viscosity of such thermoplastic elastomer expressed as Melt Flow Rate (200°C, 5 kg) measured according to standard ISO1133 is preferably above 1 g/10 min, preferably above 5 g/10 min, more preferably above 10 g/10 min although higher viscosity thermoplastic elastomers having a Melt Flow Rate (MFR) below 1 g/10 min can be used as well. The Melt Flow Rate is a measure of the ease of flow of the melt of a thermoplastic polymer. It is defined as the mass of polymer, in grams, flowing in ten minutes through a capillary of a specific diameter and length by a pressure applied via prescribed alternative gravimetric weights for alternative prescribed temperatures. Melt Flow Rate is inversely proportional to viscosity of the melt at the conditions of the test. In some embodiments, the thermoplastic elastomer may contain viscosity reducing agents such as mineral oils. The Melt Flow Rate (200°C, 5 kg) of such a component may be in the range of 20 to 40 g/10 min.

A composition according to the invention optionally comprises 2.5 to 15 wt%, preferably 5 to 10 wt% of such a thermoplastic elastomer.

In particular, good results have been obtained with thermoplastic polyurethane such as polyether or polyester based TPU with shore A hardness above 85.

The amounts of second regrind and optional thermoplastic elastomer are generally balanced so that the Resistance to Stress (RTS) of the thermoplastic material obtained from the composition of the present invention is in the range of less than 100 cm, preferably between 30 and 90 cm, more preferably between 35 and 70 cm , which is the RTS range required for the thermoplastic material to be suitable in immobilization applications such as radiation therapy and diagnostic imaging. RTS is a method to estimate the Resistance To Stretch of the sheet after activation (i.e. heating at 65°C during 3 min) by: (a) cutting a sample with standard dimensions from the extruded sheet, (b) heating the sample in hot water (65 °C) for 3 min., (c) taking out the sample from the hot water and securing one end of the sample with a clamp and providing the other end with a standard weight (135 g), (d) stretching the molten sample under the force of gravity and cooling to room temperature, (e) the value of the length of the stretched sample (cm) is the value of the RTS (cm), (f), the shorter the stretched sample, the higher the resistance to stretch is.

In case a thermoplastic elastomer is used in the present composition the weight ratio between the second regrind and the thermoplastic elastomer is generally in the range 5:1 to 1:1, preferably 3:1 to 1:1.

An additive as optionally comprised in a composition according to the invention, may be any of the standard additives like pigments, antioxidants, thickeners, processing aids, stabilizers, plasticizers, lubricants, antistatic agents, nucleating agents, flow improvers, fluorescing agents, antibacterial agents.

A composition according to the invention generally comprises 0 to 5 wt%, preferably 0.1 to 1 wt%, of total additives. Preferably a masterbatch of all additives can be prepared and added to the other ingredients of the composition. The amount of each individual additive present in the composition is preferably up to 0.5 wt%, preferably up to 0.25 wt%, more preferably up to 0.15 wt%.

A composition according to the invention may comprise less than 0,5%, preferably less than 0,3 wt%, of antisticking agent. A composition according to the invention may comprise between 0.0005 and 0.03 wt%, preferably 0.01 wt%, of pigment. A composition according to the invention may comprise between 0.05 and 1 wt%, preferably 0.25 wt%, of lubricant. A composition according to the invention may comprise between 0 and 0.5 wt%, preferably 0.05 wt%, of anti-oxidant.

The invention furthermore relates to a thermoplastic material produced from the composition according to the invention. Said thermoplastic material may be shaped into a sheet, which is suitable for use in immobilisation devices.

For the production of the thermoplastic material according to the invention, each technique which may be deemed suitable by the person skilled in the art can be used such as extrusion or injection-molding. Preferably the thermoplastic material is manufactured by extrusion(e.g. twin-screw extrusion) and extrusion-based processes and can be in the form of granules, sheets and filaments, depending of the way of making the customized product. Plastics extrusion is a well-known manufacturing process in which raw plastic is melted and formed into a continuous profile, for example films or sheets. This process starts by feeding plastic material into the barrel of the extruder. The material is gradually melted by the mechanical energy generated by turning screws and by heaters arranged along the barrel. The molten polymer is then forced into a die, which shapes the polymer into a shape that hardens during cooling.

Manufacturing thermoplastic material of the present invention by extrusion preferably takes place in a temperature range of between 200 and 230°C, which is higher than normally; this is done to avoid high shrinkage of the sheet.

A further advantage of the present invention lies in the fact that the end-use properties of the thermoplastic material are set during the extrusion process and do not require any post-processing step such as chemical crosslinking at the solid state.

Preferably the thermoplastic material is produced in the form of a sheet. The thickness of the sheet may vary within wide limits. When using the thermoplastic material for an immobilization device for immobilization of a body part, the thickness will generally vary from 1 to 4 mm, preferably from 2 to 3,2 mm. Often, a thickness of about 1.2, 1.6, 2, 2.4 or 3.2 mm is used. The skilled person will ensure in the selection of the suitable thickness that the thickness is sufficiently large to satisfy the requirements for the intended application. For immobilization devices, the thickness is usually chosen such that the bending modulus and strength end up within the required limits for that application, and that the cooling time of the thermoplastic material after being heated to its melting or softening temperature, is sufficiently long to allow forming at/around the body part to be immobilized.

The thermoplastic sheet of the present invention may be used as a solid sheet, but it may also comprise a plurality of perforations which extend through the thickness of the sheet. Such a perforated sheet provides reduced weight and sweating possibility for the patient and hence improves patient comfort. With perforations is meant that the thermoplastic sheet contains a plurality of holes, which extend throughout the material of the sheet. The perforations may be applied randomly or according to a specific pattern. The method of applying such perforations to thermoplastic sheets for use in immobilization devices is well known to the skilled person. In general, in view of optimizing the time available for moulding of the template, the degree of perforation of the thermoplastic sheet will vary between 1 and 25 %, wherein % means the % of the surface of the thermoplastic sheet occupied by perforations, in relation to the total surface of the thermoplastic sheet.

According to the invention, a sheet-shaped thermoplastic material is used for producing an immobilization device for immobilization of a body part. The material of the present invention is particularly useful in the area of radiation therapy and diagnostic imaging.

At temperatures such as temperatures of around 65°C, a thermoplastic material according to the invention is in a molten state, providing a high thermoforming behavior. In this molten state, the material is easy to shape or mold around a body part resulting in a good conformity around the body part.

Thermoplastic materials according to the invention generally have a Resistance to Stress (RTS) of between 30 and 100 cm, preferably in the range of to 35 and 70 cm

Preferably thermoplastic materials according to the invention have a bending modulus of at least above 200 MPa, preferably above 250 MPa, more preferably above 300 MPa . The bending modulus and maximum load is measured according to standard ASTM D790.

For the comfort of the patient, it is generally important that the measured vertical force, representing the mask shrinkage, after 30 min is not above 200 N and consequently does not exceed 300 N after 24h.

Preferably thermoplastic materials according to the invention offer a horizontal stability below 1,5 mm.

For the comfort of the patient, it is important that the temperature at which the material is activated/heated or moulded, is less than 90°C, preferably less than 80°C, more preferably less than 75°C, most preferably between 65 and 70°C. In general the material is activated at 65°C but left to cool down to 45-50°C before being shaped on the body of the patient.

A thermoplastic material according to the invention does not cooldown quickly after heating which offers the Radiotherapy technician enough time to get the immobilization mask out of the heating device (dry it in case of water bath) , walk to the treatment room, stretch and mold the immobilization device on the body part of the patient and finally attach it to the treatment table. In particular, the inventors have observed that the period of time which remains available for the molding of the thermoplastic material, may be at least 30 seconds, in particular at least 1 minutes or at least 1,5 minutes. In general, the molding time available, will not be longer than 100 seconds. On the other hand, the full setting/solidification of the immobilization device should not exceed 15 min, preferably around 10 min, to save clinical time and reduce patient waiting times during cooling and hardening of the mask after the molding has been finished.

The invention is illustrated by but not limited to the following Examples.

### Example 1

A thermoplastic sheet of 2 mm thickness was produced by extrusion containing the ingredients as indicated in table 1 below.

The ingredients were fed in zone 1 of Mapré twin screw extruder using 3 or 4 gravimetric feeders whereby a separate feeder was used for each ingredient, with all additives being fed together through a masterbatch. The extrusion temperature was set to 200-230°C. The screw speed was set at 250 rpm. Sheets samples were extruded using a flat die with a width of 120-150 cm.

The following properties were measured on the obtained sheet: RTS, max load and bending modulus all according to the methods described above.

Shrinkage (vertical force in N) and stability (horizontal displacement in mm) of masks made out of these sheets were measured as follows. A specially designed instrument, based on a method described in BE 1015081, incorporated herein, which can measure the shrinkage force and stability of the mask and/or to apply different fixation forces to the mask which was moulded on a dummy head was used. After activation of the mask in a water bath (3 min at 65°) or dry oven (5 min at 65°C), the fully melted (activated) mask is moulded on a dummy head on the top of the shrink & stability measuring device. The vertical force measured by the device (due to polymer crystallization) is followed as function of time. A typical curve is given below.

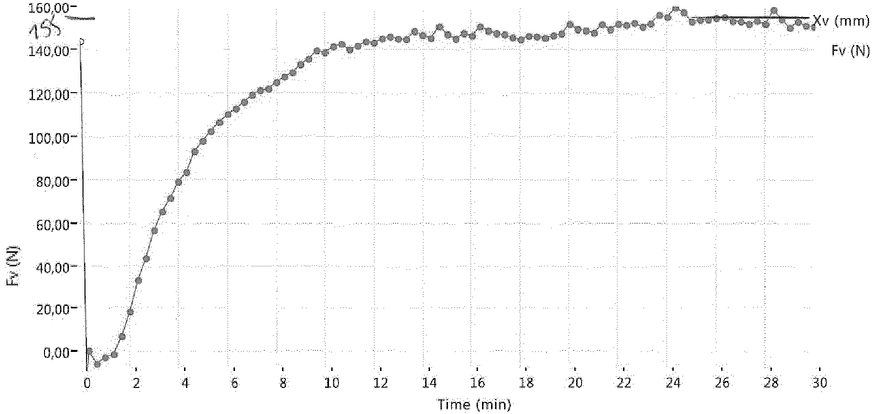

The working time corresponds to the time before the polymer crystallization (mask solidification) starts. This time is extracted from the measured curve (Vertical force vs time) and it corresponds to the time when Fv= 0. The shrinkage force corresponds to the maximum vertical force measured after 30 min and after 24h. With the stability of fixation of the mask is meant the horizontal displacement in mm of a dummy head when a particular fixation force is applied to the mask and horizontal force of 100N is applied to the dummy head.

Moulding properties and patient comfort were assessed by a product specialist in similar conditions to clinical cases. The product specialist has to score the following properties from poor to good:
With respect to Moulding (see visualisations below):
   Poor: the mask stretches too much when taken out from the activation oven (not enough melt strength) or it is too rigid and cannot be stretched. As a result it cannot be moulded correctly (un-homogenous stretching, wrinkles, tearing off, gap between the body and the mask (bad conformity)).
   Acceptable: the mask can be moulded correctly on the patient but it requires more effort from the product specialist; e.g he has to pull it a little bit more to be able to stretch or he has to work quickly as the masks start to solidify faster.
   Good: the mask can be stretched easily and can be shaped around the head and neck, it takes the anatomy shape correctly (without any wrinkles or gap (good conformity)). 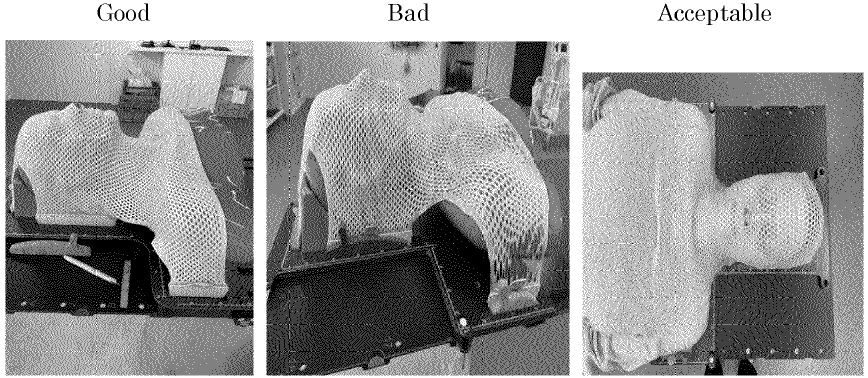
With respect to Patient comfort:
   After moulding the mask on the volunteer person, the product specialist questions the person regarding his feeling. The patient stability is also assessed (whether or not he can move). The mask comfort is assessed immediately and after 24 h.
   Accordingly, the patient comfort is ranked as a function of the volunteer's feedback.
   Poor: mask is too tight, person cannot breath correctly under the mask, high pressure on the face/nose or mask too loose, patient can move under it, resulting in unstable immobilization or mask cannot be refitted after 24 h.
   Acceptable: the pressure on the person's face is relatively high but can be tolerated by the person. No or slight movement possible under the mask hence good immobilization.
   Good: mask is not too tight, not too lose so it fits correctly. The person does not feel excessive pressure on his face . The person cannot move his head (precise immobilization).

The results are indicated in Table 1. These results show that thermoplastic material not containing second regrind (Comp 1 and Comp 5) show unsatisfactory performance.

### Example 2

A thermoplastic sheet of 2 mm thickness was produced by extrusion (according to example 1) containing the ingredients as indicated in table 2 below.

The same properties as in Example 1 were measured on the obtained sheet. The results are indicated in Table 2.

These results show that thermoplastic material containing an amount of second regrind that is too high (Comp 8 and Comp 9) show unsatisfactory performance.

**Table 1**

| **Sample No.** | | **Comp 1** | **2** | **3** | **4** | **Comp 6** | **8** | **7** |
|---|---|---|---|---|---|---|---|---|
| **Component** | **/n° formulation** | Wt % | Wt % | Wt % | Wt % | Wt % | Wt % | Wt % |
| **Poly caprolactone** | CAPA 6500 | 93,75 | | | | | | |
| **First Regrind** | Non x-linked regrind | | 62,75 | 63,75 | 72,75 | 59,75 | 63,25 | 77,75 |
| **First Regrind** | Non-x-linked regrind containing nanoclay | | | | | 30 | | |
| **Second Regrind** | X-linked regrind (RTS 100-140 cm) containing nanoclay | | 30 | | | | 30 | |
| **Second Regrind** | X-linked regrind (RTS 40-70 cm) containing nanoclay | | | | | | | 15 |
| **Second Regrind** | x-regrind from used masks (RTS 100-140 cm) | | | 30 | 20 | | | |
| **TPU** | Desmopan | 6 | 7 | 6 | 7 | 10 | 6,5 | 7 |
| **Additives** | Pigment+ Lubricant + antioxidant | 0,25 | 0,25 | 0,25 | 0,25 | 0,25 | 0,25 | 0,25 |
| **Sum Components** | | **100.00** | **100.00** | **100.00** | **100.00** | **100.00** | **100.00** | **100.00** |
| | | | | | | | | |
| **Objective measurements** | RTS (135g) | 30 | 43 | 35 | 35 | 40 | 70 | 40 |
| | Sec. Mod. (MPa) | 250 | 407 | 395 | 350 | / | 330 | 365 |
| | Max. Load (N) | / | 2,91 | 2 | 2 | / | 3,17 | 3,14 |
| | Working time (min) | 1 | 1,5 | 1,5 | 1 | 1 | 1,8 | 1,2 |
| | Vertical shrinkage force after 30 min (N) | 180 | 172 | 201 | 194 | 123 | 117 | 109 |
| | Vertical shrinkage force after 24 h (N) | 350 | 244 | 285 | 258 | 172 | 181 | 153 |
| | Horizontal stability (mm) | 1,2 | 0,63 | 0,63 | 0,64 | 1,38 | 1,36 | 1,38 |
| **Subjective measurements** | Molding properties (stretching, conforming ,...) | **Poor** | **Acceptable** | **Acceptable** | **Good** | Failed (mask tear off from the attachment the table | **Good** | **Good** |
| | Patient comfort (mask stability, tightness and shrinkage) | **Poor** | **good** | **good** | **Good** | NA | **Good** | **Good** |

**Table 2**

| **Sample No.** | | **Comp 8** | **Comp 9** | **10** | **11** | **12** |
|---|---|---|---|---|---|---|
| **Component** | **/n° formulation** | Wt % | Wt % | Wt % | Wt % | Wt % |
| **Polycaprolactone** | CAPA 6500 | 50 | | 79,75 | 59,75 | 61,75 |
| **Second Regrind** | X-linked regrind (RTS 100-140 cm) | | | 10 | | |
| **Second Regrind** | X-linked regrind (RTS 100-140 cm) containing nanoclay | 44,75 | 94,75 | | 30 | 30 |
| **TPU** | Desmopan | 5 | 5 | 10 | 10 | 8 |
| **Additives** | Pigments + Lubricants + Antioxidant | 0,25 | 0,25 | 0,25 | 0,25 | 0,25 |
| **Sum Components** | | **100.00** | **100.00** | **100.00** | **100.00** | **100.00** |
| | | | | | | |
| **Objective measurements** | RTS (135g) | More than 50 % of shrinkage in the extrusion direction) → mask size is reduced by 50 % after activation at 65°C → not useful | | 48 | 36 | 50 |
| | Sec. Mod. (MPa) | | | 295 | 315 | 450 |
| | Max. Load (N) | | | 2,37 | 1,91 | 1,15 |
| | Working time (min) | | | 1 | 1 | 1 |
| | Vertical shrinkage force after 30 min (N) | | | / | / | 122 |
| | Vertical shrinkage force after 24 h (N) | | | / | / | 186 |
| | Horizontal stability (mm) | | | / | / | 1,33 |
| **Subjective measurements** | Molding properties (stretching, conforming) | **NA** | | **good** | **Slightly poor** | **Acceptable** |
| | Patient comfort (mask stability, tightness and shrinkage) | **NA** | | **good** | **Acceptable** | **Good** |

## Claims

1. A composition comprising, based on total weight of the composition,
a. 55 to 90 wt%, preferably 60 to 80 wt%, more preferably 65 to 75 wt% of a first polymer and/or a first regrind by-product thermoplastic material derived from such a first polymer which is not chemically crosslinked, said first polymer and/or said first regrind having a melting temperature of below 100°C, preferably of between 40 and 75°C;
b. 5 to 35 wt%, preferably 10 to 30 wt%, more preferably 15 to 20 wt% of a second regrind by-product thermoplastic material derived from such a first polymer said by-product thermoplastic material being at least partially chemically crosslinked;
c. optionally 2.5 to 15 wt%, preferably 5 to 10 wt% of a thermoplastic elastomer;
d. 0 to 5 wt% of total additives.

2. The composition according to claim 1, wherein the first polymer is a polycaprolactone (PCL) polymer.

3. The composition according to claim 1 or 2, wherein the first and/or second regrind by-product thermoplastic material comprises by-product generated during the production process of the thermoplastic material e.g. by perforating some parts of the thermoplastic material or by cutting it into a desired shape.

4. The composition according to any one of the preceding claims wherein the second regrind by-product thermoplastic material contains by-product material from thermoplastic material that contain nanofillers such as nanoclay.

5. The composition according to any one of the preceding claims wherein the thermoplastic elastomer has a molecular weight above 100 000 g/mol.

6. The composition according to any of the preceding claims, wherein the thermoplastic elastomer is selected from the group consisting of thermoplastic styrenic elastomers preferably having a styrene content of 15 to 50 wt%, or Thermoplastic Polyurethanes, preferably the thermoplastic elastomer is a thermoplastic polyurethane.

7. The composition according to any one of the preceding claims wherein the amounts of second regrind by-product thermoplastic material and optional thermoplastic elastomer are such that the Resistance to Stress (RTS) of the thermoplastic material obtained from the composition is in the range 30 to 100 cm, preferably in the range 35 to 70 cm.

8. The composition according to any of the preceding claims, wherein the one or more additive is selected from the group consisting of a pigment, antioxidant, thickener, processing aid, fluorescing agent, antibacterial agent, lubricant and slipping agent.

9. A thermoplastic material made from a composition as defined in any one of claims 1-8.

10. The thermoplastic material according to claim 9 which is made by extrusion.

11. The thermoplastic material according to claim 9 or 10 which does not require a post processing step such as chemical crosslinking.

12. The thermoplastic material according to any one of claims 9 to 11 having a Resistance to Stress of 30 to 100 cm, preferably 35 to 70 cm.

13. The thermoplastic material according to any one of claims 9 to 12 which is in the form of a sheet, preferably having a thickness in the range 1 to 4 mm.

14. The use of a thermoplastic material as defined in any one of claims 9 to 13, for the manufacture of an immobilization device for immobilizing at least a portion of a body part.

15. An immobilization device for immobilizing at least a portion of a body part, wherein the immobilisation device comprises a sheet of a thermoplastic material as defined in claim 13, wherein the sheet of the thermoplastic material is shaped to conform to the body part to be immobilized.

16. Use of an immobilization device as defined in claim 15 in radiation therapy or diagnostic imaging.

17. A method for manufacturing a thermoplastic material comprising the step of extruding a composition as defined in any one of claims 1 to 8.

18. The method according to claim 17 wherein a post-processing chemical crosslinking step is not included
